# EUROPEAN PATENT SPECIFICATION

(11) **EP 0 455 432 B1**
(45) Date of publication and mention of the grant of the patent: **13.03.1996**
(21) Application number: 91303798.2
(22) Date of filing: 26.04.1991
(51) Int. Cl.: C07H 17/07, A61K 31/70, C12P 19/18, A23L 1/23, A23L 2/52, A23L 3/34, A61K 7/00

(54) **Alpha-glycosyl naringin, and its preparation and uses**
Alpha-Glycosyl-Naringin, seine Herstellung und seine Verwendung
Alpha-glycosyl naringin, et son procédé de préparation et utilisation

(30) Priority: 29.04.1990 JP 112665/90
(43) Date of publication of application: 06.11.1991
(73) Proprietor: KABUSHIKI KAISHA HAYASHIBARA SEIBUTSU KAGAKU KENKYUJO, Okayama-shi Okayama (JP)
(72) Inventor: Yoneyama, Masaru, Souja-shi, Okayama (JP); Miyake, Toshio, Okayama-shi, Okayama (JP)
(74) Representative: Pendlebury, Anthony

(56) References cited:
- EP-A- 0 325 872
- EP-A- 0 327 099
- EP-A- 0 420 376
- CHEMISCHE BERICHTE, vol. 104, no. 2, 1971, pages 473-478; G. AURNHAMMER et al.: "Synthese des 7-beta-Neohesperidosyl-4'-beta-D-glucopyranosylnaringenins, eines Flavanontriglykosids aus Citrusfrüchten"
- ACTA CHEMICA SCANDINAVICA SER. B, vol. B32, no. 10, 1978, pages 714-716; B.-G. ÖSTERDAHL: "Chemical studies on bryophytes. 20. A new branched flavonoid-O- triglycoside from Dicranum scoparium"
- CHEMICAL ABSTRACTS, vol. 91, no. 3, 16th July 1979, page 325, abstract no. 16674n, Columbus, Ohio, US; B.G. OSTERDAHL: "Chemical studies on bryophytes. 21. Flavonoid glycosides of Hedwigia ciliata", & ACTA CHEM. SCAND., SER. B. 1979, B33(2), 119-24

## Description

The present invention relates to a novel α-glycosyl naringin, and its preparation and uses.

More particularly, the present invention relates to α-glycosyl naringin wherein equimolar or more D-glucose residues are bound to naringin via the α-bond, and also to a process to prepare α-glycosyl naringin, characterized in that allowing a saccharide-transferring enzyme to act on a solution containing naringin together with an α-glucosyl saccharide to form α-glycosyl naringin, and recovering the α-glycosyl naringin.

The present invention further relates to foodstuffs including beverages and processed foods, pharmaceuticals for susceptive diseases including preventive and remedy therefor, and cosmetics including skin-refining agent and skin-whitening agent, characterized in that they all contain the α-glycosyl naringin obtainable by the process.

Naringin, whose chemical structure is given below, has been known as a vitamin P with physiological activities such as stabilization of blood vessel, prevention of hemorrhage and regulation of blood pressure, and used from ancient times in foodstuffs, pharmaceuticals and cosmetics.

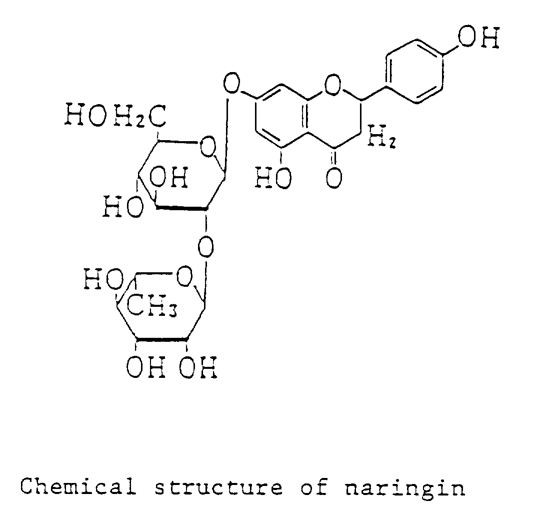

It is known that vitamin P takes part in some physiological activities of vitamin C in vivo; for example, in the hydroxylation of proline and lysine which are necessary to synthesize collagen as the main element of living connective tissues; the oxidation-reduction reaction of cytochrome C wherein Fe⁺⁺⁺ is reduced into Fe⁺⁺; and in the immunopotentiation via the increase of leukocyte. These are because vitamin P plays a significant role in the maintenance and promotion of health in living bodies.

Nowadays the use of naringin is not limited to agents which enrich vitamin P as a nutritive element, but is extending in various applications. More particularly, because of the chemical structure and physiological activities, naringin is useful as a nutritive vitamin P-enriched agent alone or in combination with one or more vitamins, for example, in foods and beverages as a bitterness-imparting agent, antioxidant, stabilizer, quality-improving agent and uv-absorbent; in pharmaceuticals for susceptive diseases such as a preventive and remedy for viral diseases, bacterial diseases, circulatory diseases and malignant tumors; and in cosmetics such as skin-refining and skin-whitening agents, i.e. a stabilizer, antioxidant, uv-absorbent and melanin-formation inhibitory agent.

Naringin is, however, hardly soluble in water only about 1g in 1 liter of water or about 0.1 w/v % at 25°C. This renders its practical use very difficult.

Chemische Berichte, vol 104, no. 2, 1971 discloses on pages 473-478 synthesis of the glycoside, 7-β-Neohesperidosyl-4'-(β-D-glucopyranosyl) naringenin. The glycoside was prepared by the condensation of phloracetophenone 4-β-neohesperidoside with 4-hydroxylbenzaldehyde 4-β-D-glucopyranoside, followed by cyclisation to form the β-glycoside.

Accordingly, the realization of a naringin derivative which is free from the drawbacks of conventional naringin and its derivatives, in particular, superior in water-solubility and free from toxicity while retaining the bitterness inherent to naringin, and exhibits a desired physiological activity in vivo has been in strong demand.

The present invention aims to overcome the drawbacks of prior art. We investigated novel naringin derivatives by utilizing a biochemical procedure.

As the result, we found that a novel α-glycosyl naringin is formed by allowing a saccharide-transferring enzyme to act on a solution containing naringin together with an α-glucosyl saccharide, as well as that the α-glycosyl naringin retains the bitterness of naringin per se and is superior in water-solubility, free from toxicity, and readily hydrolyzable in vivo to exhibit the physiological activity inherent to naringin. Furthermore, we established its preparation and uses in foodstuffs, pharmaceuticals for susceptive diseases, and cosmetics. Thus, we accomplished the present invention.

We also found that the α-glucosyl naringin formed by the saccharide-transfer reaction is easily purified by allowing a reaction mixture to contact with a synthetic macroporous resin, and utilizing the difference in absorbability thereto.

Thus, we confirmed that the process according to the invention completely overcomes the drawback of prior art, and extremely facilitates the commercialization of α-glycosyl naringin.

The invention will now be described further by way of example only with reference to the drawings in which:

FIG.1 shows an infrared absorption spectrum of α-glycosyl naringin specimen [II] as an example of the present invention.

FIG.2 shows an infrared absorption spectrum of intact naringin as the control.

The present invention will be explained in detail hereinafter , by way of example only.

The naringin usable in the invention shall not be limited to those in a highly-purified form. For example, mixtures with flavonoid glycosides such as citronin, hesperidin and rutin, and intact and partially-purified extracts from plant tissues are suitable, as long as they contain naringin.

Citrus fruits including those in immature form are suitable for the plant tissue, as well as rinds of citrus fruits.

The α-glucosyl saccharides usable in the invention are those which permit a saccharide-transferring enzyme to form α-glycosyl naringin from naringin. For example, partial starch hydrolysates such as amylose, dextrin, cyclodextrin and maltooligosaccharide, liquefied starch, and gelatinized starch are suitably chosen.

Consequently to facilitate the formation of α-glycosyl naringin, it is recommendable to choose for particular saccharide-transferring enzyme an α-glucosyl saccharide having an adequate susceptivity thereto.

For example, in the case of using α-glucosidase (EC 3.2.1.20) as the saccharide-transferring enzyme, maltooligosaccharides such as maltose, maltotriose and maltotetraose are suitable, as well as partial starch hydrolysates having a DE (dextrose equivalent) in the range of about 10-70. When cyclomaltodextrin glucanotransferase (EC 2.4.1.19) is used as the saccharide-transferring enzyme, gelatinized starches having a DE of below 1 and partial starch hydrolysates having a DE up to about 60 are suitable, as well as cyclodextrins. When α-amylase (EC 3.2.1.1) is used as the saccharide-transferring enzyme, gelatinized starches having a DE of below 1 and dextrins and partial starch hydrolysates having a DE up to about 30 are suitable.

The concentration of such an α-glucosyls accharide during the reaction is set to a level which is about 0.5-100-fold higher, preferably, about 2-20-fold higher than that of naringin.

During the reaction, desirably, naringin is kept at the possible highest concentration. For example, such a concentration is feasible with a high-naringin content suspension and solution both having a naringin content of about 1 w/v % or higher, preferably, about 2-20.0 w/v %. The latter solution is obtainable by dissolving naringin by heating or dissolving naringin at an alkaline pH exceeding 7.0.

The saccharide-transferring enzymes usable in the present invention are those which form α-glucosyl naringin without decomposing naringin when allowed to act on a solution which contains naringin together with an α-glucosyl saccharide having an adequate susceptivity to the enzyme.

Examples of such a saccharide-transferring enzyme are α-glucosidases derived from animal and plant tissues such as pig liver and buckwheat seed, and from a culture obtainable by cultivating in a nutrient culture medium microorganisms including molds and yeasts, for example, those of the genera Mucor, Penicillium and Saccharomyces; cyclomaltodextrin glucanotransferases derived from a culture of bacteria such as those of the genera Bacillus and Klebsiella; and α-amylases s derived from a culture of bacteria and fungi such as those of the genera Bacillus and Aspergillus.

Such a saccharide-transferring enzyme should not necessarily be purified prior to its use, as long as it fulfills the above requirements. Generally, the present invention is feasible with a crude enzyme.

If necessary, saccharide-transferring enzymes can be purified by conventional method, prior to its use. Of course, commercialized saccharide-transferring enzymes can be used in the invention.

The amount of saccharide-transferring enzyme and reaction time are closely dependent each other. With an economical viewpoint, saccharide-transferring enzyme is used in an amount which completes the reaction within about 5-80 hours.

Immobilized saccharide-transferring enzymes can be suitably used batchwise and in continuous manner.

In order to prevent the degradation of naringin in a reaction solution, such enzymatic reaction is desirably carried out under a light-shielding-, anaerobic- and slightly alkaline-condition if possible.

The resultant reaction mixture containing α-glycosyl naringin may be prepared into final products without no further special treatment. Usually, the reaction mixture is filtered and concentrated into a syrupy product which is, if necessary, dried and prepared into a powdery product.

These products are favorably usable as a highly-safe natural bitterness-imparting agent, antioxidant, stabilizer, quality-improving agent, preventive, remedy and uv-absorbent in foods, beverages, tobaccos, feeds, pet foods, pharmaceuticals for susceptive diseases, cosmetics and plastics, as well as a vitamin P-enriching agent.

In case that a purified α-glycosyl naringin product is needed, α-glycosyl naringin and contaminants including α-glucosyl saccharides are separated by utilizing the difference in adsorbability to a synthetic macroporous resin.

The wording "synthetic macroporous resin" as referred to in the invention means non-ionic, macroporous, synthetic resins which provide a large adsorptive area, such as styrene-divinylbenzen copolymer, phenol-formaldehyde resin, acrylic resin and methacrylate resins. Examples of such resins are "Amberlite XAD-1", "Amberlite XAD-2", "Amberlite XAD-4", "Amberlite XAD-7", "Amberlite XAD-8", "Amberlite XAD-11" and "Amberlite XAD-12", products of Rohm & Haas Co., Philadelphia, USA; "Diaion HP-10", "Diaion HP-20", "Diaion HP-30", "Diaion HP-40" and "Diaion HP-50", products of Mitsubishi Chemical Industries Ltd., Tokyo, Japan; and "Imac Syn-42", "Imac Syn-44" and "Imac Syn-46", products of Industrie de Maatshappily activate N.V., Amsterdam, Netherlands.

The purification process according to the invention contains the step of applying a reaction mixture containing α-glycosyl naringin, for example, to a column of a synthetic macroporous resin so that the column adsorbs the α-glycosyl rutin and a relatively small amount of the remaining naringin, while large amounts of α-glucosyl and water-soluble saccharides flow out through the column without causing adsorption.

If necessary, after completion of the saccharide-transfer reaction but before treatment with a synthetic macroporous resin, the reaction mixture can be treated by one or more methods; for example, a method wherein the reaction mixture is heated and the insolubilized substances are removed by filtration; another method wherein the reaction mixture is treated, for example, with either magnesium alumino silicate hydrate or magnesium aluminate to adsorb the proteinaceous substances for their removal; and one another method wherein the reaction mixture is deionized with an ion-exchange resin (H - or OH-form).

A column of a synthetic macroporous resin on which α-glycosyl naringin and a relatively small amount of the remaining naringin have been specifically adsorbed are washed with a diluted alkali or water, and then applied with a relatively small amount of an organic solvent or mixture with water, for example, aqueous methanol and aqueous ethanol. Thus, the α-glycosyl naringin first elutes, while the intact naringin is eluted by continuing the application or increasing the concentration of the organic solvent.

The obtained eluate rich in α-glycosyl naringin is distilled to remove the organic solvent, and concentrated to an adequate level. Thus, one can obtain a syrupy product mainly composed of α-glycosyl naringin. Subsequent drying and pulverization of the product yield a powdery product mainly composed of α-glycosyl naringin.

The elution operation using organic solvents simultaneously regenerates synthetic macroporous resins, and this enables their repeated use.

The purification process using synthetic macroporous resins is characterized in that it can remove, in addition to α-glucosyl and water-soluble saccharides, other contaminants including water-soluble salts.

The α-glycosyl naringin thus obtained is characterized by:
(1) It is extremely higher in water-solubility than intact naringin.
(2) It is higher in resistance to light and stability than intact naringin.
(3) It has substantially the same bitterness as intact naringin.
(4) It is hydrolyzable into naringin and glucose by the in vivo enzyme system to exhibit the physiological activity inherent to naringin, in particular, vitamin P activity. Combination with vitamin C augments the physiological activities of both vitamins.
(5) When an α-glycosyl naringin product additionally contains an α-glucosyl saccharide, the α-glycosyl naringin exhibits its inherent activities, while the α-glucosyl saccharide exhibits shape-imparting, filling and sweetening activities. A product free from α-glucosyl saccharide exhibits the activity of α-glycosyl naringin without causing substantial shape-imparting and increase in quantity.

Because of these, α-glycosyl naringin can be favorably incorporated as a bitterness-imparting agent, antioxidant, stabilizer, quality-improving agent, preventive and remedy for susceptive diseases such as viral diseases, bacterial diseases, circulatory diseases and malignant tumors, and uv-absorbent in foods, beverages, tobaccos, feeds, pet foods, pharmaceuticals for susceptive diseases, cosmetics such as skin-refining agents and skin-whitening agents, and plastics, as well as in agents which are directed to enrich a highly-safe, natural vitamin P.

Since α-glycosyl naringin is highly resistant to acid and heat, and well harmonizes with various substances which taste sour, salty, delicious and astringent, it can be favorably incorporated in foods and beverages in general and tobaccos, for example, seasonings, Japanese-style confectioneries, Western-style confectioneries, ice creams, sherbets, soft drinks, alcoholic drinks, spreads, pastes, pickles, pickled products, bottled products, canned products, meat products, fish meat products, milk products, egg products, processed vegetables, processed fruits and cereals.

Particularly, the bitterness of α-glycosyl naringin can be favorably imparted to citrus, cocoa, coffee, chocolate, teas, crude drugs (or galenicas) and flavoring agents thereof in order to improve their taste- and flavor-qualities. Furthermore, α-glycosyl naringin can be advantageously used to improve its taste and flavor in combination with one or more sweeteners such as those derived from plants, for example, stevioside, α-glycosyl stevioside, rebaudioside A, glycyrrhizin, α-glycosyl glycyrrhizin and dihydrochalcone; amino acid sweeteners, for example, glycine, alanine and L-aspartylphenylalanine methyl ester; and saccharide sweeteners, for example, sucrose, starch syrup, glycosyl sucrose, glucose, isomerized saccharide, fructose, honey, maltose, sorbitol, maltitol and lactose. In addition, when α-glycosyl raringin is used in foodstuffs such as fruit juice and gellies thereof, the foodstuffs do not become turbid and cloudy because α-glycosyl naringin prevents the crystalization and sedimentation of flavonoids compounds in the fruit juice. Furthermore, α-glycosyl naringin can be favorably incorporated in feeds and pet foods for domestic animals and poultries including pet animals such as honey bee, silkworm and pet fish in order to enrich them with vitamin P and also to improve their taste qualities.

In addition to the use as a uv-absorbent and deterioration-preventing agent for plastics, α-glycosyl naringin can be favorably incorporated in tobaccos, pharmaceuticals including preventive and remedy for susceptive diseases, and cosmetics including skin-refining agent and skin-whitening agent in solid, paste or liquid; for example, cigar, cigarette, troche, cod-liver oil drop, vitamin compound, oral refreshing agent, cachou, gargle, intubation nutrient, crude drug (or galenicals), internal medicine, injection, dentifrice, lipstick, lip cream and sun-screening.

The wording "susceptive diseases" as referred to in the invention means those which are prevented and/or treated with α-glycosyl naringin; for example, viral diseases, bacterial diseases, traumatic diseases, immunopathies, rheumatism, diabetes, circulatory diseases and malignant tumors. The shape and form of pharmaceuticals for susceptive diseases can be freely chosen to meet to their final use; for example, liquid pharmaceuticals such as nebula, collyrium, collunarium, collutory and injection, paste pharmaceuticals such as ointment, cataplasm and cream, and solid pharmaceuticals such as powder, granule, capsule and tablet. In the preparation of such a pharmaceutical, one or more ingredients, for example, remedy, biologically-active substance, antibiotic, adjuvant, filler, stabilizer, coloring agent and flavoring agent, can be suitably used in combination, if necessary.

The dose is adequately changed dependent on the α-glycosyl naringin content, administration route and administration frequency; usually, in the range of about 0.001-10.0g/day/adult as α-glycosyl naringin.

Cosmetics can be prepared similarly as in pharmaceuticals.

In use, α-glycosyl naringin is incorporated in products by conventional method, for example, mixing, kneading, dissolving, soaking, permeating, spreading, applying, spraying and injecting, before completion of their processing.

The following experiments will illustrate α-glycosyl naringin according to the invention.

### Experiment 1

### Preparation of α-glycosyl naringin

### Experiment 1(1)

### Saccharide-transfer reaction

One part by weight of naringin and 6 parts by weight of dextrin (DE 20) were added with 50 parts by weight of water, and the mixture was dissolved by heating, added with 20 units/g dextrin of cyclomaltodextrin glucanotransferase from Bacillus stearothermophilus commercialized by Hayashibara Biochemical Laboratories, Inc., Okayama, Japan, allowed to react for 18 hours while keeping the mixture at pH 6.0 and 70°C, and heated to inactivate the enzyme. Thus, an α-glycosyl naringin-containing liquid was obtained.

### Experiment 1(2)

### Purification

A reaction mixture obtained by the method in Experiment 1(1) was filtered, and the filtrate was applied to a column of "HP-10", a synthetic macroporous resin commercialized by Mitsubishi Chemical Industries Ltd., Tokyo, Japan, at a flow rate of SV 2. The column was then washed with water, and applied with 50 v/v % aqueous ethanol, after which the eluate was concentrated in vacuo to remove the ethanol, and pulverized to obtain a pale yellow α-glycosyl naringin specimen [I] in the yield of about 130% against the weight of the starting naringin, on the dry solid basis (d.s.b.).

### Experiment 1(3)

### Hydrolysis by amylase

A small portion of an α-glycosyl naringin specimen [I] obtained by the method in Experiment 1(2) was dissolved in water to 1 w/v %, and the solution was added with 100 units/g specimen of glucoamylase (EC 3.2.1.3) commercialized by Seikagaku Kogyo Co., Ltd., Tokyo, Japan, and allowed to react for 5 hours while keeping the solution at pH 5.0 and 55°C. The reaction mixture was heated to inactivate the remaining enzyme and filtered, after which the filtrate was applied to a column of "HP-10", a synthetic macroporous resin commercialized by Mitsubishi Chemical Industries Ltd., Tokyo, Japan, at a flow rate of SV 2. As the result, the column adsorbed the α-glycosyl naringin and remaining naringin, while such as glucose and salts flew out through the column without causing adsorption. The column was then washed by applying thereto water, and further applied with an aqueous ethanol having a stepwisely increasing concentration to recover an α-glycosyl naringin-rich fraction which was then concentrated in vacuo and pulverized to obtain a pale yellow α-glycosyl naringin specimen [II] in the yield of about 70% against the weight of the starting naringin, d.s.b.

Another portion of the α-glycosyl naringin specimen [I] was hydrolyzed similarly as above except that the glucoamylase was replaced with β-amylase (EC 3.2.1.2) commercialized by Seikagaku Kogyo Co., Ltd., Tokyo, Japan, and the resultant hydrolysate was purified, concentrated and pulverized similarly as above to obtain a pale yellow α-glycosyl naringin specimen [III] in the yield of about 70% against the weight of the starting naringin, d.s.b.

### Experiment 2

### Characterization of α-glycosyl naringin

### Experiment 2(1)

### Improvement of water-solubility

An α-glycosyl naringin-containing solution prepared by the method in Experiment 1(1) using the saccharide-transfer reaction, and a control solution which had been prepared similarly except that the enzyme was inactivated by heating prior to its use were allowed to stand at 4°C for 2 days. As the result, in the control the sedimentation of naringin led to a white turbidity, while the solution containing α-glycosyl naringin left transparent.

Accordingly, the α-glycosyl naringin formed by the saccharide-transfer reaction has an extremely improved water-solubility.

### Experiment 2(2)

### Solubility in solvents

α-Glycosyl naringin specimens were readily soluble in water, 0.1N sodium hydroxide and 0.1N hydrochloric acid; slightly soluble in methanol and ethanol; and insoluble in ether benzene and chloroform.

### Experiment 2(3)

### Taste quality

Intact naringin and α-glycosyl naringin specimens [I], [II] and [III] were prepared into their respective aqueous solutions (1 mM), and their taste qualities were compared. It was found that either of the specimens [I], [II] and [III] favorably retained the bitterness of intact naringin and exhibited the same level of bitterness as that of intact naringin.

### Experiment 2(4)

### uv-Absorption spectrum

α-Glycosyl naringin specimens were prepared into their respective aqueous solutions (pH 6.0), and determined their uv-absorption spectra. Either of the specimens [I], [II] and [III] exhibited absorption peaks at about 211, 282 and 329nm as intact naringin.

### Experiment 2(5)

### Infrared absorption spectrum

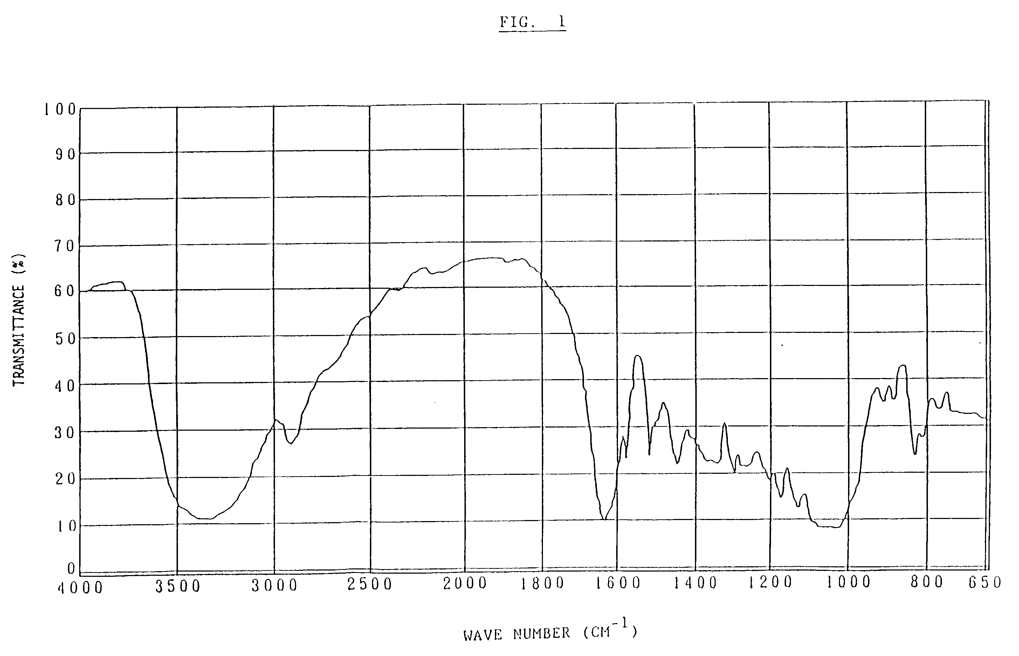

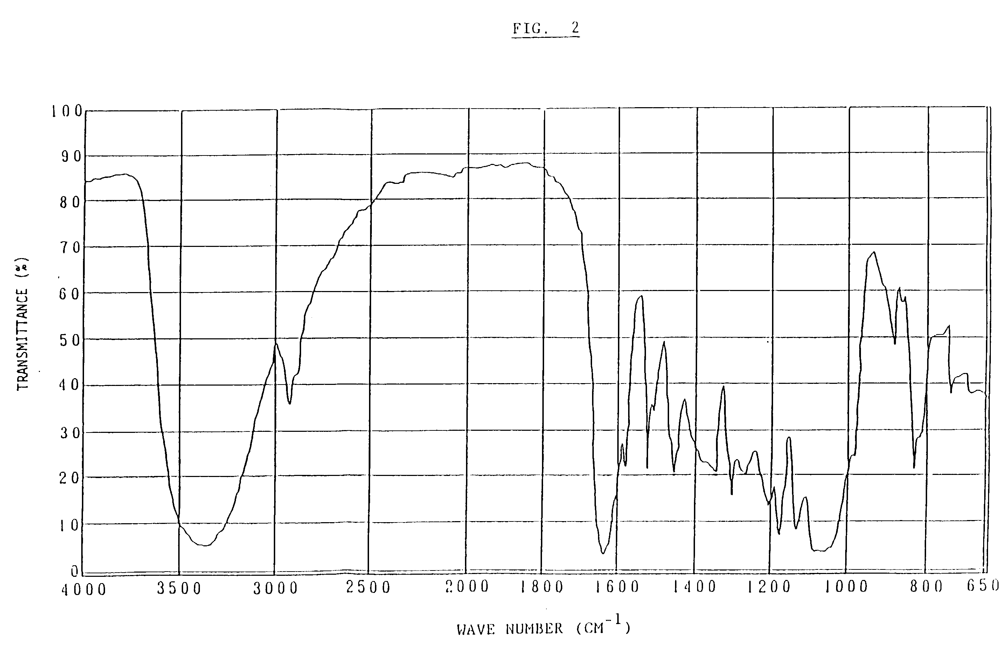

The infrared absorption spectra of α-glycosyl naringin specimens were determined by the KBr tablet method. FIGs.1 and 2 show the infrared absorption spectra of the specimen [II] and intact naringin as the control.

### Experiment 2(6)

### Stability against hydrolysis

(a) α-Glycosyl naringin specimens are hydrolyzable by α-glucosidase (EC 3.2.1.20) derived from pig liver into naringin and D-glucose.
(b) Not hydrolyzable by β-glucosidase.

### Experiment 2(7)

### Thin-layer chromatography

(a) Analytic procedure
   Thin-layer plate: "Kieselgel 60 F254" commercialized by Merck & Co., Inc., Rahway, New Jersey, USA
   Developing solvent: ethyl acetate:formic acid:water =11:2:3
   Color-developing agent: 10 w/w % aqueous sulfuric acid solution
(b) Results
   Analysis of the α-glycosyl naringin specimens revealed that the specimen [I] exhibited spots at Rf 0.68, 0.49, 0.38, 0.21 and other spots near the starting point in addition to a spot at Rf 0.79; the specimen [II], a spot at Rf 0.68; and the specimen [III], spots at Rf 0.68 and 0.49.

The above described physicochemical properties suggest that the substance exhibiting a spot at Rf 0.68 in the specimens [I], [II] and [III] is α-glucosyl naringin wherein 1 mole of D-glucose residue is bound to 1 mole naringin via the α-bond; the substance exhibiting a spot at Rf 0.49 in the specimens [I] and [III], α-maltosyl naringin wherein 2 moles of D-glucose is bound to 1 mole of naringin via the α-bond; and the substance exhibiting a plurality of spots at not higher than Rf 0.38 in the specimen [I], α-maltooligosyl naringin wherein 3 moles or more D-glucose residues are bound to naringin via the α-bond.

As described above, the α-glycosyl naringin according to the invention wherein equimolar or more D-glucose residues are bound to naringin via the α-bond is a novel, satisfactorily-high water-soluble naringin derivative which is hydrolyzable in vivo by α-glucosidase to exhibit the physiological activity inherent to naringin when ingested.

### Experiment 3

### Acute toxicity

An α-glycosyl naringin specimen [I], prepared by the method in Experiment 1(2), was orally administered to 7 week-old dd mice for acute toxicity test. As the result, no mouse died when adninistered with up to 5g of the specimen, and higher dose was difficult.

These confirmed that the specimen was extremely low in toxicity. An α-glycosyl naringin specimen [II], prepared by the method in Experiment 1(3), was tested similarly as above to obtain the same result, confirming that the toxicity of this specimen was extremely low.

The following Examples A and Examples B will illustrate the preparation and uses of α-glycosyl naringin according to the invention.

### Example A-1

### α-Glycosyl naringin

One part by weight of naringin and 4 parts by weight of dextrin (DE 10) were dissolved in 10 parts by weight of water, and the resultant solution was dissolved by heating, cooled to 75°C, and promptly added with 20 units/g dextrin of cyclomaltodextrin glucanotransferase derived from Bacillus stearothermophilus commercialized by Hayashibara Biochemical Laboratories, Inc., Okayama, Japan, and allowed to react for 24 hours under stirring conditions while keeping the solution at pH 5.5 and 75°C. Thin-layer chromatographic analysis of the reaction mixture revealed that about 65% of the naringin was converted into α-glycosyl naringins such as α-glucosyl naringin, α-maltosyl naringin, α-maltotriosyl naringin and α-maltotetraosyl naringin. Thereafter, the reaction mixture was heated to inactivate the remaining enzyme and filtered, after which the filtrate was deionized and purified with ion exchanges (H- and OH-forms), and concentrated in usual manner to obtain a syrupy α-glycosyl naringin product additionally containing α-glucosyl saccharides in the yield of about 85% against the weight of the starting materials, d.s.b.

The product is favorably usable as a highly-safe, natural bitterness-imparting agent, antioxidant, stabilizer, quality-improving agent, preventive, remedy and uv-absorbent in foods, beverages, tobaccos, feeds, pet foods, pharmaceuticals for susceptive diseases, cosmetics and plastics, as well as in vitamin P-enriching agents.

### Example A-2

### α-Glucosyl naringin

One part by weight of a syrupy α -glycosyl naringin product additionally containing α-glucosyl saccharides, prepared in accordance with the method in Example A-1 with a slight modification, was dissolved in 4 parts by weight of water, and the solution was added with 100 units/g syrup solid of glucoamylase (EC 3.2.1.3) commercialized by Seikagaku Kogyo, Co., Ltd., Tokyo, Japan, and allowed to react at 50°C for 5 hours. Thin-layer chromatographic analysis of the reaction mixture revealed that the α-glycosyl naringin was converted into α-glucosyl naringin.

Thereafter, the reaction mixture was heated to inactivate the remaining enzyme and filtered, after which the filtrate was applied to a column of "HP-10", a synthetic macroporous resin commercialized by Mitsubishi Chemical Industries Ltd., Tokyo, Japan, at a flow rate of SV 2. As the result, the column adsorbed the α-glucosyl naringin and remaining naringin both present in the reaction mixture, while glucose and salts flew out through the column without causing adsorption. The column was then washed by applying thereto water, and further applied with an aqueous ethanol having a stepwisely increasing concentration to recover an α-glucosyl naringin-rich fraction which was then concentrated in vacuo and pulverized to obtain a powdery α-glucosyl naringin in the yield of about 55% against the weight of the starting naringin, d.s.b.

Acid hydrolysis of the α-glucosyl naringin led to the formation of 1 mole of rhamnose and 2 moles of D-glucose per 1 mole naringenin, while an α-glucosidase, obtained by the extraction from pig liver and partial purification, hydrolyzed α-glucosyl naringin into naringin and D-glucose.

The product is favorably usable as a bitterness-imparting agent, antioxidant, stabilizer, quality-improving agent, preventive, remedy and uv-absorbent in foods, beverages, tobaccos, pharmaceuticals for susceptive diseases, and cosmetics,as well as in an agent directed to enrich a highly-purified, readily water-soluble vitamin P.

### Example A-3

### α-Glycosyl naringin

One part by weight of naringin and 10 parts by weight of dextrin (DE 8) were dissolved in 20 parts by weight of water by heating. The resultant solution was cooled to 70°C, added with 30 units/g dextrin of cyclomaltodextrin glucanotransferase, and allowed to react for 40 hours under stirring conditions while keeping the mixture at pH 6.0 and 65°C.

Thin-layer chromatographic analysis of the reaction mixture revealed that about 75% of the naringin was converted into α-glycosyl naringin.

Thereafter, the reaction mixture was heated to inactivate the remaining enzyme and filtered, after which the filtrate was applied to a column of "XAD-7", a synthetic macroporous resin commercialized by Rohm and Haas Co., Philadelphia, USA, at a flow rate of SV 1.5.

As the result, the column adsorbed the α-glycosyl naringin and remaining naringin both present in the reaction mixture, while dextrin, oligosaccharides and salts flew out through the column without causing adsorption.

The column was then washed by applying thereto water, and further applied with 50 v/v % aqueous methanol to elute the α-glycosyl naringin and naringin which were then concentrated and pulverized to obtain a powdery α-glycosyl naringin in the yield of about 110% against the weight of the starting naringin, d.s.b.

The product is favorably usable as a highly-safe, natural bitterness-imparting agent, antioxidant, stabilizer, quality-improving agent, preventive, remedy and uv-absorbent in foods, beverages, tobaccos, feeds, pet foods, pharmaceutical for susceptive diseases, cosmetics and plastics, as well as in the use directed to enrich a readily water-soluble vitamin P.

### Example A-4

### α-Glycosyl naringin

### Example A-4(1)

### Preparation of α-glucosidase

Mucor javanicus IFO 4570 was inoculated and cultivated at 30°C for 44 hours under aeration-agitation conditions in 500 parts by weight of a liquid culture medium which contained water together with 4 w/v % maltose, 0.1 w/v % potassium phosphate monobasic, 0.1 w/v % ammonium nitrate, 0.05 w/v % magnesium sulfate, 0.05 w/v % potassium chloride, 0.2 w/v % polypeptone and 1 w/v % calcium carbonate which had been sterilised by heating and sterilely added to the water immediately before the innoculation. After completion of the cultivation, the mycelia was collected from the culture, added with 500 parts by weight of 4M urea in 0.5M acetate buffer (pH 5.3) per 48 parts by weight of the wet mycelia, allowed to stand at 30°C for 40 hours and centrifuged. The supernatant was dialyzed against flowing water overnight, added with ammonium sulfate to 0.9 saturation, and allowed to stand at 4°C overnight, after which the resultant sediment was collected, suspended in 50 parts by weight of 0.01M acetate buffer (pH 5.3) and centrifuged. The supernatant was recovered and used as an α-glucosidase specimen.

### Example A-4(2)

### Preparation of α-glycosyl naringin

Three parts by weight of naringin and 20 parts by weight of dextrin (DE 30) were dissolved in 50 parts by weight of water by heating, and the resultant solution was cooled to 55°C, promptly added with 10 parts by weight of an α-glucosidase prepared by the method in Example A-4(1), and allowed to react for 40 hours under stirring conditions while keeping the solution at pH 6.0 and 55°C.

Thin-layer chromatographic analysis of the reaction mixture revealed that about 50% of the naringin was converted into α-glycosyl naringin.

Thereafter, the reaction mixture was purified, concentrated and pulverized similarly as in Example A-3 to obtain a powdery α-glycosyl naringin product in the yield of about 95% against the weight of the starting naringin, d.s.b.

Similarly as the product in Example A-3, the product is feasible as a highly-safe, natural bitterness-imparting agent, antioxidant, stabilizer, quality-improving agent, preventive, remedy and uv-absorbent, as well as an agent directed to enrich a readily water-soluble vitamin P.

### Example B-1

### Hard candy

Fifteen hundred parts by weight of "MABIT®", a hydrogenated maltose syrup commercialized by Hayashibara Shoji, Inc., Okayama, Japan, was heated, concentrated to a moisture content below about 2%, and mixed with 1 part by weight of an α-glycosyl naringin powder obtained by the method in Example A-3 and an adequate amount of caramel and coffee flavor, after which the mixture was molded and packaged in usual manner to obtain a hard candy.

The product is a low-cariogenic and low-caloric coffee candy enriched with vitamin P and bitterness.

### Example B-2

### "Fuki-no-mizuni" (Boiled bop rhubarb)

Fresh bog rhubargs were pared, cut into short sticks, soaked in a diluted saline for hours, and boiled down in a liquid which contained an α-glycosyl naringin syrup obtained by the method in Example A-1 and a green coloring composition prepared by mixing an α-glycosyl rutin and "Aoiro Ichi-go" (Blue no.1), a green coloring agent, to obtain a freshly green "fuki-no-mizuni".

The product is enriched with a satisfiable natural bitterness and favorably used as a material for Japanese traditional cuisines.

### Example B-3

### "Gyuhi" ( starch paste)

One part by weight of waxy rice starch was mixed with 1.2 parts by weight of water, and the mixture was mixed to homogeneity with 1.5 parts by weight of sucrose, 0.7 parts by weight of "SUNMALT®", a crystalline β-maltose commercialized by Hayashibara Co., Ltd., Okayama, Japan, 0.3 parts by weight of starch syrup and 0.02 parts by weight of an α-glycosyl naringin syrup obtained by the method in Example A-1 while gelatinizing by heating. Thereafter, the resultant was molded and packaged in usual manner to obtain "gyuhi".

The product is a tasteful Japanese-style confectionery enriched with a satisfiable natural bitterness, which looks like "kibi-dango" (millet dumpling).

### Example B-4

### Mixed sweetener

A mixed sweetener was obtained by mixing 100 parts by weight of honey, 50 parts by weight of isomerized sugar, 1 part by weight of "α-G sweet", an α-glycosyl stevioside, commercialized by Toyo Sugar Refining Co., Ltd., Tokyo, Japan, and 0.02 parts by weight of an α-glycosyl naringin powder obtained by the method in Example A-4.

The product is suitable for health food because the product is a vitamin P-enriched sweetener with an about 2-fold sweetening power of sucrose.

### Example B-5

### Cream filling

A cream filling was obtained by mixing to homogeneity in usual manner 1,200 parts by weight of "FINETOSE®", a crystalline α-maltose commercialized by Hayashibara Co., Ltd., Okayama, Japan, 1,000 parts by weight of shortening, 50 parts by weight of cocoa mass, 3 parts by weight of an α-glycosyl naringin powder obtained by the method in Example A-3, and 1 part by weight of lecithin.

The product is a vitamin P- and bitterness-enriched cream filling which is excellent in taste, flavor, melting and biting properties.

### Example B-6

### Tablet

Twenty parts by weight of ascorbic acid wasmixed to homogeneity with 13 parts by weight of crystalline β-maltose, 4 parts by weight of corn starch and 3 parts by weight of an α-glucosyl naringin obtained by the method in Example A-2, and the resultant was tabletted with a 20R punch, diameter of 12mm.

The product is an easily swallowable vitamin composition containing L-ascorbic acid and α-glucosyl naringin, wherein the ascorbic acid is excellently stable.

### Example B-7

### Capsule

Ten parts by weight of calcium acetate monohydrate, 50 parts by weight of magnesium L-lactate trihydrate, 57 parts by weight of maltose, 20 parts by weight of an α-glucosyl naringin obtained by the method in Example A-2, and 12 parts by weight of a γ-cyclodextrin inclusion compound containing 20% eicosapentaenoic acid were mixed to homogeneity, and the mixture was fed to a granulator, and then encapsulated in gelatine to obtain capsules, 150mg each.

The product is favorably usable as a high-quality blood cholesterol lowering agent, immunopotentiator and skin-refining agent in preventive and remedy for susceptive diseases, as well as in foodstuffs directed to the maintenance and promotion of health.

### Example B-8

### Ointment

One part by weight of sodium acetate trihydrate, 4 parts by weight of DL-calcium lactate and 10 parts by weight of glycerine were mixed to homogeneity, and the mixture was added to another mixture of 50 parts by weight of vaseline, 10 parts by weight of vegetable wax, 10 parts by weight of lanolin, 14.5 parts by weight of sesame oil, 1 part by weight of an α-glycosyl naringin obtained by the method in Example A-4 and 0.5 parts by weight of peppermint oil, and the resultant mixture was mixed to homogeneity to obtain an ointment.

The product is favorably usable as a high-quality sun-screening, skin-refining agent, skin-whitening agent and promoter for healing injury and burn.

### Example B-9

### Injection

An α-glucosyl naringin obtained by the method in Example A-2 was dissolved in water, purified and filtered in usual manner to obtain a pyrogen-free solution which was then distributed to 20ml glass vials to give an α-glucosyl naringin content of 50mg, dried in vacuo and sealed to obtain the captioned product.

The product is intramuscularly and intravenously administrable alone or in combination with vitamins and minerals. The product requires no cold storage, and exhibits an excellently high solubility in saline when in use.

### Example B-10

### Injection

Six parts by weight of sodium chloride, 0.3 parts by weight of potassium chloride, 0.2 parts by weight of calcium chloride, 3.1 parts by weight of sodium lactate, 45 parts by weight of maltose and 1 part of an α-glucosyl naringin obtained by the method in Example A-2 were dissolved in 1,000 parts by weight of water, purified and filtered in usual manner, after which 250ml aliquots of the pyrogen-free solution were distributed to sterilized plastic vessels to obtain the captioned product.

The product is capable of supplementing, in addition to vitamin P, calorie and minerals, therefore is favorably usable in the restoration of health during and after suffering from diseases.

### Example B-11

### Intubation nutrient

Twenty four gram aliquots of a compound consisting of 20 parts by weight of crystalline α-maltose, 1.1 parts by weight of glycine, 0.18 parts by weight of sodium glutamate, 1.2 parts by weight of sodium chloride, 1 part by weight of citric acid, 0.4 parts by weight of calcium lactate, 0.1 part by weight of magnesium carbonate, 0.01 part by weight of an α-glycosyl naringin obtained by the method in Example A-3, 0.01 part by weight of thiamine and 0.01 part by weight of riboflavin were packed in laminated aluminum small bags, and heat-sealed to obtain the captioned product.

In use, one bag of the product is dissolved in about 300-500ml of water, and the solution is favorably usable as an intubation nutrient directed to oral and parenteral administration to the nasal cavity, stomach and intestine.

### Example B-12

### Bath liquid

A bath liquid was obtained by mixing 21 parts of DL-sodium lactate, 8 parts by weight of sodium pyruvate, 5 parts by weight of an α-glycosyl naringin obtained by the method in Example A-1 and 40 parts by weight of ethanol with 26 parts by weight of refined water and appropriate amounts of coloring agent and flavoring agent.

The product is suitable for a skin-refining agent and skin-whitening agent, which is diluted by 100-10,000-folds in bath water when in use. In this case, bath water is replaceable with cleansing liquid, astringent and moisture liquid.

### Example B-13

### Milky lotion

One half part by weight of polyoxyethylene behenyl ether, 1 part by weight of polyoxyethylene sorbitol tetraoleate, 1 part by weight of oil-soluble glyceryl monostearate, 0.5 parts by weight of pyruvic acid, 0.5 parts by weight of behenyl alcohol, 1 part by weight of avocado oil, 1 part by weight of an α-glycosyl naringin obtained by the method in Example A-3 and appropriate amounts of vitamin E and antiseptic were dissolved by heating in usual manner, and the solution was added with 1 part by weight of L-sodium lactate, 5 parts by weight of 1,3-butylene glycol, 0.1 part by weight of carboxy-vinyl polymer and 85.3 parts by weight of refined water, emulsified with a homogenizer, added with an appropriate amount of flavoring agent, and mixed by stirring to obtain the captioned product.

The product is favorably usable as a high-quality sun-screening, skin-refining agent and skin-whitening agent.

### Example B-14

### Cosmetic cream

Two parts by weight of polyoxyethylene glycol mono-stearate, 5 parts by weight of self-emulsifying glycerine monostearate, 2 parts by weight of an α-glucosyl naringin powder obtained by the method in Example A-2, 1 part by weight of liquid paraffin, 10 parts by weight of glyceryl trioctanate and an appropriate amount of antiseptic were dissolved in usual manner by heating, and the mixture was added with 2 parts by weight of L-lactic acid, 5 parts by weight of 1,3-butylene glycol and 66 parts by weight of refined water, emulsified with a homogenizer, added with an appropriate amount of flavoring agent, and mixed by stirring to obtained the captioned product.

The product is favorably usable as a high-quality sunscreen cream, skin-refining agent and skin-whitening agent.

### [Effect of the invention]

As described above, the α-glycosyl naringin of the invention, wherein equimolar or more D-glucose residues are bound to naringin via the α-bond, has a bitterness, and is superior in water-solubility, free of toxicity, and readily hydrolyzable in vivo into naringin and D-glucose to exhibits the physiological activity inherent to naringin.

The α-glycosyl naringin is economically superior and easily commercializable because it is easily produceable by a biochemical procedure wherein a saccharide-transferring enzyme is allowed to act on a solution containing naringin together with an α-glucosyl saccharide.

Furthermore, we found that naringin can be allowed to react at an increased initial concentration and this facilitates the formation of α-glycosyl naringin at a high concentration. It was also found that in the purification of a reaction mixture, the α-glycosyl naringin can be recovered by allowing the reaction mixture to contact with a synthetic macroporous resin. These render the large-scale production of α-glycosyl naringin very easy.

Since the α-glycosyl naringin thus obtained is characterized in that it exhibits a satisfactorily-high water-solubility, light-resistance, stability and physiological activity, it is favorably usable as a bitterness-imparting agent, antioxidant, stabilizer, preventive, remedy, uv-absorbent and deterioration-preventing agent in foods, beverages, tobaccos, feeds, pet foods, pharmaceuticals for susceptive diseases, cosmetics including skin-refining agent and skin-whitening agent, and plastics, as well as in a highly-safe, natural vitamin P-enriching agent.

Accordingly, the present invention is extremely significant in food, beverage, cosmetic, pharmaceutical and plastic industries in view of the establishment of industrial-scale production and practical uses for α-glycosyl naringin.

## Claims (Claims for the following Contracting State(s): AT, BE, CH, LI, DE, DK, FR, GB, IT, LU, NL, SE)

1. α-Glycosyl naringin wherein equimolar or more D-glucose residues are bound to naringin via the α-bond.

2. α-glycosyl naringin according to claim 1, which is α-glucosyl naringin.

3. A process for preparing α-glycosyl naringin, comprising:
allowing a saccharide-transferring enzyme to act on a liquid containing naringin together with an α-glucosyl saccharide to form α-glycosyl naringin; and
recovering the α-glycosyl naringin.

4. A process according to claim 3, wherein the recovering step comprises:
purifying the liquid by allowing it to contact with a synthetic macroporous resin; and
recovering the α-glycosyl naringin.

5. A process according to claim 3 or claim 4, wherein said saccharide-transferring enzyme is a member selected from the group consisting of α-glucosidase (EC 3.2.1.20), cyclomaltodextrin glucanotransferase (EX 2.4.1.19), and α-amylase (EC 3.2.1.1).

6. A process according to any one of claims 3 to 5, wherein said liquid contains at least about 1 w/v % naringin.

7. A process according to any one of claims 3 to 6, wherein said α-glucosyl saccharide is a member selected from the group consisting of partial starch hydrolysate, liquefied starch, gelatinized starch, and mixtures thereof.

8. A process according to any one of claims 3 to 7, wherein the concentration of said α-glucosyl saccharide is 0.5-100-fold higher than that of naringin.

9. A process according to any one of claims 3 to 8, wherein said liquid is in suspension.

10. A process according to any one of claims 3 to 8, wherein said liquid is in solution.

11. A process according to claim 10, wherein said liquid is obtainable by dissolving naringin by heating.

12. A process according to claim 10, wherein said liquid is obtainable by dissolving naringin at an alkaline pH exceeding 7.0.

13. Foods and beverages, which contain α-glycosyl naringin.

14. Foods and beverages according to claim 13, wherein said α-glycosyl naringin is an α-glucosyl naringin.

15. Foods and beverages according to claim 13, wherein said α-glycosyl naringin is prepared by the process of any one of claims 3 to 12.

16. Foods and beverages according to any one of claims 13 to 15, which additionally contain vitamin C.

17. A pharmaceutical for susceptive diseases, which contains together with a pharmaceutically-acceptable carrier an α-glycosyl naringin as the effective ingredient.

18. A pharmaceutical according to claim 17, wherein said α-glycosyl naringin is α-glucosyl naringin.

19. A pharmaceutical according to claim 17, wherein said α-glycosyl naringin is prepared by the process of any one of claims 3 to 12.

20. A pharmaceutical according to any one of claims 17 to 19, which additionally contains vitamin C.

21. A cosmetic which contains as the effective ingredient an α-glycosyl naringin.

22. A cosmetic according to claim 21, wherein said α-glycosyl naringin is α-glucosyl naringin.

23. A cosmetic according to claim 21, wherein said α-glycosyl naringin is prepared by the process of any one of claims 3 to 12.

24. A cosmetic according to any one of claims 21 to 23, which additionally contains vitamin C.

25. An α-glycosyl naringin obtainable by the process of any one of claims 3 to 12.

## Claims (Claims for the following Contracting State(s): ES)

1. A process for preparing α-glycosyl naringin, comprising:
allowing a saccharide-transferring enzyme to act on a liquid containing naringin together with an α-glucosyl saccharide to form α-glycosyl naringin; and
recovering the α-glycosyl naringin.

2. A process according to claim 1, wherein the recovering step comprises:
purifying the liquid by allowing it to contact with a synthetic macroporous resin; and
recovering the α-glycosyl naringin.

3. A process according to claim 1 or claim 2, wherein said saccharide-transferring enzyme is a member selected from the group consisting of α-glucosidase (EC 3.2.1.20), cyclomaltodextrin glucanotransferase (EX 2.4.1.19), and α-amylase (EC 3.2.1.1).

4. A process according to any one of claims 1 to 3, wherein said liquid contains at least about 1 w/v % naringin.

5. A process according to any one of claims 1 to 4, wherein said α-glucosyl saccharide is a member selected from the group consisting of partial starch hydrolysate, liquefied starch, gelatinized starch, and mixtures thereof.

6. A process according to any one of claims 1 to 5, wherein the concentration of said α-glucosyl saccharide is 0.5-100-fold higher than that of naringin.

7. A process according to any one of claims 1 to 6, wherein said liquid is in suspension.

8. A process according to any one of claims 1 to 6, wherein said liquid is in solution.

9. A process according to claim 8, wherein said liquid is obtainable by dissolving naringin by heating.

10. A process according to claim 8, wherein said liquid is obtainable by dissolving naringin at an alkaline pH exceeding 7.0.

11. Foods and beverages, which contain α-glycosyl naringin.

12. Foods and beverages according to claim 11, wherein said α-glycosyl naringin is an α-glucosyl naringin.

13. Foods and beverages according to claim 11, wherein said α-glycosyl naringin is prepared by the process of any one of claims 1 to 10.

14. Foods and beverages according to any one of claims 11 to 13, which additionally contain vitamin C.

15. A process for preparing a pharmaceutical for susceptive diseases, which process comprises mixing with a pharmaceutically-acceptable carrier an α-glycosyl naringin as the effective ingredient.

16. A process according to claim 15, wherein said α-glycosyl naringin is α-glucosyl naringin.

17. A process according to claim 15, wherein said α-glycosyl naringin is prepared by the process of any one of claims 1 to 10.

18. A process according to any one of claims 15 to 17, which additionally comprises mixing vitamin C with the pharmaceutically-acceptable carrier and/or the α-glycosyl naringin.

19. A process for preparing a cosmetic, which process comprises mixing with a cosmetically-acceptable carrier an α-glycosyl naringin as the effective ingredient.

20. A process according to claim 19, wherein said α-glycosyl naringin is α-glucosyl naringin.

21. A process according to claim 20, wherein said α-glycosyl naringin is prepared by the process of any one of claims 1 to 10.

22. A process according to any one of claims 19 to 21, which additionally comprises mixing vitamin C with the cosmetically-acceptable carrier and/or the α-glycosyl naringin.

## Claims (Claims for the following Contracting State(s): GR)

1. α-Glycosyl naringin wherein equimolar or more D-glucose residues are bound to naringin via the α-bond.

2. α-glycosyl naringin according to claim 1, which is α-glucosyl naringin.

3. A process for preparing α-glycosyl naringin, comprising:
allowing a saccharide-transferring enzyme to act on a liquid containing naringin together with an α-glucosyl saccharide to form α-glycosyl naringin; and
recovering the α-glycosyl naringin.

4. A process according to claim 3, wherein the recovering step comprises:
purifying the liquid by allowing it to contact with a synthetic macroporous resin; and
recovering the α-glycosyl naringin.

5. A process according to claim 3 or claim 4, wherein said saccharide-transferring enzyme is a member selected from the group consisting of α-glucosidase (EC 3.2.1.20), cyclomaltodextrin glucanotransferase (EX 2.4.1.19), and α-amylase (EC 3.2.1.1).

6. A process according to any one of claims 3 to 5, wherein said liquid contains at least about 1 w/v % naringin.

7. A process according to any one of claims 3 to 6, wherein said α-glucosyl saccharide is a member selected from the group consisting of partial starch hydrolysate, liquefied starch, gelatinized starch, and mixtures thereof.

8. A process according to any one of claims 3 to 7, wherein the concentration of said α-glucosyl saccharide is 0.5-100-fold higher than that of naringin.

9. A process according to any one of claims 3 to 8, wherein said liquid is in suspension.

10. A process according to any one of claims 3 to 8, wherein said liquid is in solution.

11. A process according to claim 10, wherein said liquid is obtainable by dissolving naringin by heating.

12. A process according to claim 10, wherein said liquid is obtainable by dissolving naringin at an alkaline pH exceeding 7.0. 0

13. Foods and beverages, which contain α-glycosyl naringin.

14. Foods and beverages according to claim 13, wherein said α-glycosyl naringin is an α-glucosyl naringin.

15. Foods and beverages according to claim 13, wherein said α-glycosyl naringin is prepared by the process of any one of claims 3 to 12.

16. Foods and beverages according to any one of claims 13 to 15, which additionally contain vitamin C.

17. A process for preparing a pharmaceutical for susceptive diseases, which process comprises mixing with a pharmaceutically-acceptable carrier an α-glycosyl naringin as the effective ingredient.

18. A process according to claim 17, wherein said α-glycosyl naringin is α-glucosyl naringin.

19. A process according to claim 17, wherein said α-glycosyl naringin is prepared by the process of any one of claims 3 to 12.

20. A process according to any one of claims 17 to 19, which additionally comprises mixing vitamin C with the pharmaceutically-acceptable carrier and/or the α-glycosyl naringin.

21. A cosmetic which contains as the effective ingredient an α-glycosyl naringin.

22. A cosmetic according to claim 21, wherein said α-glycosyl naringin is α-glucosyl naringin.

23. A cosmetic according to claim 21, wherein said α-glycosyl naringin is prepared by the process of any one of claims 3 to 12.

24. A cosmetic according to any one of claims 21 to 23, which additionally contains vitamin C.

25. An α-glycosyl naringin obtainable by the process of any one of claims 3 to 12.

## Patentansprüche (Patentansprüche für folgende(n) Vertragsstaat(en): AT, BE, CH, LI, DE, DK, FR, IT, LU, NL, SE)

1. α-Glycosylnaringin, in welchem äquimolare oder mehr D-Glucosereste über die α-Bindung an Naringin gebunden sind.

2. α-Glycosylnaringin gemäß Anspruch 1, welches α-Glucosylnaringin ist.

3. Verfahren zur Herstellung von α-Glycosylnaringin, umfassend:
Einwirkenlassen eines Saccharid-übertragenden Enzyms auf eine Flüssigkeit, welche Naringin enthält, zusammen mit einem α-Glucosylsaccharid, um α-Glycosylnaringin zu bilden; und
Gewinnen des α-Glycosylnaringins.

4. Verfahren gemäß Anspruch 3, wobei der Gewinnungsschritt umfaßt:
Reinigen der Flüssigkeit, indem sie mit einem synthetischen makroporösen Harz kontaktieren gelassen wird; und
Gewinnen des α-Glycosylnaringins

5. Verfahren gemäß Anspruch 3 oder Anspruch 4, wobei das Saccharid-übertragende Enzym aus der Gruppe von α-Glucosidase (EC 3.2.1.20), Cydomaltodextringlucanotransferase (EX 2.4,1.19) und α-Amylase (EC 3.2.1.1) gewählt ist.

6. Verfahren gemäß einem der Ansprüche 3 bis 5, wobei die Flüssigkeit mindestens etwa 1 Gew./Vol.-% Naringin enthält.

7. Verfahren gemäß einem der Ansprüche 3 bis 6, wobei das α-Glucosylsaccharid aus der Gruppe von Stärketeilhydrolysat, verflüssigte Stärke, gelatinisierte Stärke und Gemischen davon gewählt ist.

8. Verfahren gemäß einem der Ansprüche 3 bis 7, wobei die Konzentration an α-Glucosylsaccharid 0,5-100fach höher als jene des Naringins ist.

9. Verfahren gemäß einem der Ansprüche 3 bis 8, wobei die Flüssigkeit in Suspension ist.

10. Verfahren gemäß einem der Ansprüche 3 bis 8, wobei die Flüssigkeit in Lösung ist.

11. Verfahren gemäß Anspruch 10, wobei die Flüssigkeit durch Auflösen von Naringin durch Erwärmen erhältlich ist.

12. Verfahren gemäß Anspruch 10, wobei die Flüssigkeit erhältlich ist, indem Naringin bei einem alkalischen pH über 7,0 aufgelöst wird.

13. Lebensmittel und Getränke, welche α-Glycosylnaringin enthalten.

14. Lebensmittel und Getränke nach Anspruch 13, wobei das α-Glycosylnaringin ein α-Glucosylnaringin ist.

15. Lebensmittel und Getränke gemäß Anspruch 13, wobei das α-Glycosylnaringin mit dem Verfahren eines der Ansprüche 3 bis 12 hergestellt ist.

16. Lebensmittel und Getränke nach einem der Ansprüche 13 bis 15, welche zusätzlich Vitamin C enthalten.

17. Pharmazeutikum für darauf ansprechende Krankheiten, welches ein α-Glycosylnaringin als den wirksamen Inhaltsstoff zusammen mit einem pharmazeutisch akzeptablen Träger enthält.

18. Pharmazeutikum gemäß Anspruch 17, wobei das α-Glycosylnaringin α-GlucosyInaringin ist.

19. Pharmazeutikum gemäß Anspruch 17, wobei das α-Glycosylnaringin mit dem Verfahren eines der Ansprüche 3 bis 12 hergestellt ist.

20. Pharmazeutikum gemäß einem der Ansprüche 17 bis 19, welches zusätzlich Vitamin C enthält.

21. Kosmetikum, welches ein α-Glycosylnaringin als den wirksamen Inhaltsstoff enthält.

22. Kosmetikum gemäß Anspruch 21, wobei das α-Glycosylnaringin α-Glucosylnaringin ist.

23. Kosmetikum gemäß Anspruch 21, wobei das α-Glycosylnaringin mit dem Verfahren eines der Ansprüche 3 bis 12 hergestellt ist.

24. Kosmetikum gemäß einem der Ansprüche 21 bis 23, welches zusätzlich Vitamin C enthält.

25. α-Glycosylnaringin, welches mit dem Verfahren eines der Ansprüche 3 bis 12 erhältlich ist.

## Patentansprüche (Patentansprüche für folgende(n) Vertragsstaat(en): ES)

1. Verfahren zur Herstellung von α-Glycosylnaringin, umfassend:
Einwirkenlassen eines Saccharid-übertragenden Enzyms auf eine Flüssigkeit, welche Naringin enthält, zusammen mit einem α-Glucosylsaccharid, um α-Glycosylnaringin zu bilden; und
Gewinnen des α-Glycosylnaringins.

2. Verfahren gemäß Anspruch 1, wobei der Gewinnungsschritt umfaßt:
Reinigen der Flüssigkeit, indem sie mit einem synthetischen makroporösen Harz kontaktieren gelassen wird; und
Gewinnen des α-Glycosylnaringins.

3. Verfahren gemäß Anspruch 1 oder Anspruch 2, wobei das Saccharid-übertragende Enzym aus der Gruppe von α-Glucosidase (EC 3.2.1.20), Cyclomaltodextringlucanotransferase (EX 2.4.1.19) und α-Amylase (EC 3.2.1.1) gewählt ist.

4. Verfahren gemäß einem der Ansprüche 1 bis 3, wobei die Flüssigkeit mindestens etwa 1 Gew./Vol.-% Naringin enthält.

5. Verfahren gemäß einem der Ansprüche 1 bis 4, wobei das α-Glucosylsaccharid aus der Gruppe von Stärketeilhydrolysat, verflüssigte Stärke, gelatinisierte Stärke und Gemischen davon gewählt ist.

6. Verfahren gemäß einem der Ansprüche 1 bis 5, wobei die Konzentration an α-Glucosylsaccharid 0,5-100fach höher als jene des Naringins ist.

7. Verfahren gemäß einem der Ansprüche 1 bis 6, wobei die Flüssigkeit in Suspension ist.

8. Verfahren gemäß einem der Ansprüche 1 bis 6, wobei die Flüssigkeit in Lösung ist.

9. Verfahren gemäß Anspruch 8, wobei die Flüssigkeit durch Auflösen von Naringin durch Erwärmen erhältlich ist.

10. Verfahren gemäß Anspruch 8, wobei die Flüssigkeit erhältlich ist, indem Naringin bei einem alkalischen pH über 7,0 aufgelöst wird.

11. Lebensmittel und Getränke, welche α-Glycosylnaringin enthalten.

12. Lebensmittel und Getränke nach Anspruch 11, wobei das α-Glycosylnaringin ein α-Glucosylnaringin ist.

13. Lebensmittel und Getränke gemäß Anspruch 11, wobei das α-Glycosylnaringin mit dem Verfahren eines der Ansprüche 1 bis 10 hergestellt ist.

14. Lebensmittel und Getränke nach einem der Ansprüche 11 bis 13, welche zusätzlich Vitamin C enthalten.

15. Verfahren zur Herstellung eines Pharmazeutikums für darauf ansprechende Krankheiten, welches Verfahren ein Mischen eines α-Glycosylnaringins als den wirksamen Inhaltsstoff mit einem pharmazeutisch akzeptablen Träger umfaßt.

16. Verfahren gemäß Anspruch 15, wobei das α-Glycosylnaringin α-Glucosylnaringin ist.

17. Verfahren gemäß Anspruch 15, wobei das α-Glycosylnaringin mit dem Verfahren eines der Ansprüche 1 bis 10 hergestellt ist.

18. Verfahren gemäß einem der Ansprüche 15 bis 17, welches zusätzlich ein Mischen von Vitamin C mit dem pharmazeutisch akzeptablen Träger und/oder dem α-Glycosylnaringin umfaßt.

19. Verfahren zur Herstellung eines Kosmetikums, welches Verfahren ein Mischen eines α-Glycosylnaringins als den wirksamen Inhaltsstoff mit einem kosmetisch akzeptablen Träger umfaßt.

20. Verfahren gemäß Anspruch 19, wobei das α-Glycosylnaringin α-Glucosylnaringin ist.

21. Verfahren gemäß Anspruch 20, wobei das α-Glycosylnaringin mit dem Verfahren eines der Ansprüche 1 bis 10 hergestellt ist.

22. Verfahren gemäß einem der Ansprüche 19 bis 21, welches Verfahren zusätzlich ein Mischen von Vitamin C mit dem kosmetisch akzeptablen Träger und/oder dem α-Glycosylnaringin umfaßt.

## Patentansprüche (Patentansprüche für folgende(n) Vertragsstaat(en): GR)

1. α-Glycosylnaringin, in welchem äquimolare oder mehr D-Glucosereste über die α-Bindung an Naringin gebunden sind.

2. α-Glycosylnaringin gemäß Anspruch 1, welches α-Glucosylnaringin ist.

3. Verfahren zur Herstellung von α-Glycosylnaringin, umfassend:
Einwirkenlassen eines Saccharid-übertragenden Enzyms auf eine Flüssigkeit, welche Naringin enthält, zusammen mit einem α-Glucosylsaccharid, um α-Glycosylnaringin zu bilden; und
Gewinnen des α-Glycosylnaringins.

4. Verfahren gemäß Anspruch 3, wobei der Gewinnungsschritt umfaßt:
Reinigen der Flüssigkeit, indem sie mit einem synthetischen makroporösen Harz kontaktieren gelassen wird; und
Gewinnen des α-Glycosylnaringins.

5. Verfahren gemäß Anspruch 3 oder Anspruch 4, wobei das Saccharid-übertragende Enzym aus der Gruppe von α-Glucosidase (EC 3.2.1.20), Cyclomaltodextringlucanotransferase (EX 2.4.1.19) und α-Amylase (EC 3.2.1.1) gewählt ist.

6. Verfahren gemäß einem der Ansprüche 3 bis 5, wobei die Flüssigkeit mindestens etwa 1 Gew./Vol.-% Naringin enthält.

7. Verfahren gemäß einem der Ansprüche 3 bis 6, wobei das α-Glucosylsaccharid aus der Gruppe von Stärketeilhydrolysat, verflüssigte Stärke, gelatinisierte Stärke und Gemischen davon gewählt ist.

8. Verfahren gemäß einem der Ansprüche 3 bis 7, wobei die Konzentration an α-Glucosylsaccharid 0,5-100fach höher als jene des Naringins ist.

9. Verfahren gemäß einem der Ansprüche 3 bis 8, wobei die Flüssigkeit in Suspension ist.

10. Verfahren gemäß einem der Ansprüche 3 bis 8, wobei die Flüssigkeit in Lösung ist.

11. Verfahren gemäß Anspruch 10, wobei die Flüssigkeit durch Auflösen von Naringin durch Erwärmen erhältlich ist.

12. Verfahren gemäß Anspruch 10, wobei die Flüssigkeit erhältlich ist, indem Naringin bei einem alkalischen pH über 7,0 aufgelöst wird.

13. Lebensmittel und Getränke, welche α-Glycosylnaringin enthalten.

14. Lebensmittel und Getränke nach Anspruch 13, wobei das α-Glycosylnaringin ein α-Glucosylnaringin ist.

15. Lebensmittel und Getränke gemäß Anspruch 13, wobei das α-Glycosylnaringin mit dem Verfahren eines der Ansprüche 3 bis 12 hergestellt ist.

16. Lebensmittel und Getränke nach einem der Ansprüche 13 bis 15, welche zusätzlich Vitamin C enthalten.

17. Verfahren zur Herstellung eines Pharmazeutikums für darauf ansprechende Krankheiten, welches Verfahren ein Mischen eines α-Glycosylnaringins als den wirksamen Inhaltsstoff mit einem pharmazeutisch akzeptablen Träger umfaßt.

18. Verfahren gemäß Anspruch 17, wobei das α-Glycosylnaringin α-Glucosylnaringin ist.

19. Verfahren gemäß Anspruch 17, wobei das α-Glycosylnaringin mit dem Verfahren eines der Ansprüche 3 bis 12 hergestellt ist.

20. Verfahren gemäß einem der Ansprüche 17 bis 19, welches zusätzlich ein Mischen von Vitamin C mit dem pharmazeutisch akzeptablen Träger und/oder dem α-Glycosylnaringin umfaßt.

21. Kosmetikum, welches ein α-Glycosylnaringin als den wirksamen Inhaltsstoff enthält.

22. Kosmetikum gemäß Anspruch 21, wobei das α-Glycosylnaringin α-Glucosylnaringin ist.

23. Kosmetikum gemäß Anspruch 21, wobei das α-Glycosylnaringin mit dem Verfahren eines der Ansprüche 3 bis 12 hergestellt ist.

24. Kosmetikum gemäß einem der Ansprüche 21 bis 23, welches zusätzlich Vitamin C enthält.

25. α-Glycosylnaringin, welches mit dem Verfahren eines der Ansprüche 3 bis 12 erhältlich ist.

## Revendications (Revendications pour l'(les) Etat(s) contractant(s) suivant(s): AT, BE, CH, LI, DE, DK, FR, IT, LU, NL, SE)

1. Naringine α-glycosylique dans laquelle des résidus de D-glucose au moins équimolaires sont liés à la naringine par l'intermédiaire de la liaison α.

2. Naringine α-glycosylique selon la revendication 1, qui est une naringine α-glucosylique.

3. Procédé de préparation d'une naringine α-glycosylique, comportant :
l'action d'une saccharide-transférase sur un liquide contenant de la naringine ainsi qu'un saccharide α-glucosylique pour former une naringine α-glycosylique ; et
la récupération de la naringine α-glycosylique.

4. Procédé selon la revendication 3, dans lequel l'étape de récupération comporte :
la purification du liquide en le mettant en contact avec une résine synthétique macroporeuse ; et
la récupération de la naringine α-glycosylique.

5. Procédé selon la revendication 3 ou la revendication 4, dans lequel ladite saccharide-transférase est un membre choisi dans le groupe constitué par l'α-glucosidase (EC 3.2.1.20), la cyclomaltodextrine-glucanotransférase (EC 2.4.1.19) et l'α-amylase (EC 3.2.1.1).

6. Procédé selon l'une quelconque des revendications 3 à 5, dans lequel ledit liquide contient au moins 1% pds/v environ de naringine.

7. Procédé selon l'une quelconque des revendications 3 à 6, dans lequel ledit saccharide α-glucosylique est un membre choisi dans le groupe constitué par l'hydrolysat partiel d'amidon, l'amidon liquéfié, l'amidon gélatinisé et leurs mélanges.

8. Procédé selon l'une quelconque des revendications 3 à 7, dans lequel la concentration dudit saccharide α-glucosylique est 0,5 à 100 fois supérieure à celle de la naringine.

9. Procédé selon l'une quelconque des revendications 3 à 8, dans lequel ledit liquide est en suspension.

10. Procédé selon l'une quelconque des revendications 3 à 8, dans lequel ledit liquide est en solution.

11. Procédé selon la revendication 10, dans lequel on peut obtenir ledit liquide par dissolution de la naringine en chauffant.

12. Procédé selon la revendication 10, dans lequel on peut obtenir ledit liquide par dissolution de la naringine à un pH alcalin excédant 7,0.

13. Aliments et boissons, qui contiennent de la naringine α-glycosylique.

14. Aliments et boissons selon la revendication 13, où ladite naringine α-glycosylique est une naringine α-glucosylique.

15. Aliments et boissons selon la revendication 13, où ladite naringine α-glycosylique est préparée par le procédé de l'une quelconque des revendications 3 à 12.

16. Aliments et boissons selon l'une quelconque des revendications 13 à 15, qui contiennent en outre de la vitamine C.

17. Produit pharmaceutique pour des maladies susceptibles, qui contient ainsi qu'un porteur acceptable du point de vue pharmaceutique, une naringine α-glycosylique comme ingrédient efficace.

18. Produit pharmaceutique selon la revendication 17, dans lequel ladite naringine α-glycosylique est une naringine α-glucosylique.

19. Produit pharmaceutique selon la revendication 17, dans lequel ladite naringine α-glycosylique est préparée par le procédé de l'une quelconque des revendications 3 à 12.

20. Produit pharmaceutique selon l'une quelconque des revendications 17 à 19, qui contient en outre de la vitamine C.

21. Cosmétique qui contient comme ingrédient efficace une naringine α-glycosylique.

22. Cosmétique selon la revendication 21, dans lequel ladite naringine α-glycosylique est une naringine α-glucosylique.

23. Cosmétique selon la revendication 21, dans lequel ladite naringine α-glycosylique est préparée par le procédé de l'une quelconque des revendications 3 à 12.

24. Cosmétique selon l'une quelconque des revendications 21 à 23, qui contient en outre de la vitamine C.

25. Naringine α-glycosylique que l'on peut obtenir par le procédé de l'une quelconque des revendications 3 à 12.

## Revendications (Revendications pour l'(les) Etat(s) contractant(s) suivant(s): ES)

1. Procédé de préparation d'une naringine α-glycosylique, comportant :
l'action d'une saccharide-transférase sur un liquide contenant de la naringine ainsi qu'un saccharide α-glucosylique pour former une naringine α-glycosylique ; et
la récupération de la naringine α-glycosylique.

2. Procédé selon la revendication 1, dans lequel l'étape de récupération comporte :
la purification du liquide en le mettant en contact avec une résine synthétique macroporeuse ; et
la récupération de la naringine α-glycosylique.

3. Procédé selon la revendication 1 ou la revendication 2, dans lequel ladite saccharide-transférase est un membre choisi dans le groupe constitué par l'α-glucosidase (EC 3.2.1.20), la cyclomaltodextrine-glucanotransférase (EC 2.4.1.19) et l'α-amylase (EC 3.2.1.1).

4. Procédé selon l'une quelconque des revendications 1 à 3, dans lequel ledit liquide contient au moins 1% pds/v environ de naringine.

5. Procédé selon l'une quelconque des revendications 1 à 4, dans lequel ledit saccharide α-glucosylique est un membre choisi dans le groupe constitué par l'hydrolysat partiel d'amidon, l'amidon liquéfié, l'amidon gélatinisé et leurs mélanges.

6. Procédé selon l'une quelconque des revendications 1 à 5, dans lequel la concentration dudit saccharide α-glucosylique est 0,5 à 100 fois supérieure à celle de la naringine.

7. Procédé selon l'une quelconque des revendications 1 à 6, dans lequel ledit liquide est en suspension.

8. Procédé selon l'une quelconque des revendications 1 à 6, dans lequel ledit liquide est en solution.

9. Procédé selon la revendication 8, dans lequel on peut obtenir ledit liquide par dissolution de la naringine en chauffant.

10. Procédé selon la revendication 8, dans lequel on peut obtenir ledit liquide par dissolution de la naringine à un pH alcalin excédant 7,0.

11. Aliments et boissons, qui contiennent de la naringine α-glycosylique.

12. Aliments et boissons selon la revendication 11, où ladite naringine α-glycosylique est une naringine α-glucosylique.

13. Aliments et boissons selon la revendication 11, où ladite naringine α-glycosylique est préparée par le procédé de l'une quelconque des revendications 1 à 10.

14. Aliments et boissons selon l'une quelconque des revendications 11 à 13, qui contiennent en outre de la vitamine C.

15. Procédé de préparation d'un produit pharmaceutique pour des maladies susceptibles, lequel procédé comporte le mélange avec un porteur acceptable du point de vue pharmaceutique d'une naringine α-glycosylique comme ingrédient efficace.

16. Procédé selon la revendication 15, dans lequel ladite naringine α-glycosylique est une naringine α-glucosylique.

17. Procédé selon la revendication 15, dans lequel ladite naringine α-glycosylique est préparée par le procédé de l'une quelconque des revendications 1 à 10.

18. Procédé selon l'une quelconque des revendications 15 à 17, qui comporte en outre le mélange de vitamine C avec le support acceptable du point de vue pharmaceutique et/ou la naringine α-glycosylique.

19. Procédé de préparation d'un cosmétique, lequel procédé comporte le mélange avec un support acceptable du point de vue cosmétique d'une naringine α-glycosylique comme ingrédient efficace.

20. Procédé selon la revendication 19, dans lequel ladite naringine α-glycosylique est une naringine α-glucosylique.

21. Procédé selon la revendication 20, dans lequel ladite naringine α-glycosylique est préparée par le procédé de l'une quelconque des revendications 1 à 10.

22. Procédé selon l'une quelconque des revendications 19 à 21, qui comporte en outre le mélange de vitamine C avec le support acceptable du point de vue cosmétique et/ou la naringine α-glycosylique.

## Revendications (Revendications pour l'(les) Etat(s) contractant(s) suivant(s): GR)

1. Naringine α-glycosylique dans laquelle des résidus de D-glucose au moins équimolaires sont liés à la naringine par l'intermédiaire de la liaison α.

2. Naringine α-glycosylique selon la revendication 1, qui est une naringine α-glucosylique.

3. Procédé de préparation d'une naringine α-glycosylique, comportant :
l'action d'une saccharide-transférase sur un liquide contenant de la naringine ainsi qu'un saccharide α-glucosylique pour former une naringine α-glycosylique ; et
la récupération de la naringine α-glycosylique.

4. Procédé selon la revendication 3, dans lequel l'étape de récupération comporte :
la purification du liquide en le mettant en contact avec une résine synthétique macroporeuse ; et
la récupération de la naringine α-glycosylique.

5. Procédé selon la revendication 3 ou la revendication 4, dans lequel ladite saccharide-transférase est un membre choisi dans le groupe constitué par l'α-glucosidase (EC 3.2.1.20), la cyclomaltodextrine-glucanotransférase (EC 2.4.1.19) et l'α-amylase (EC 3.2.1.1).

6. Procédé selon l'une quelconque des revendications 3 à 5, dans lequel ledit liquide contient au moins 1% pds/v environ de naringine.

7. Procédé selon l'une quelconque des revendications 3 à 6, dans lequel ledit saccharide α-glucosylique est un membre choisi dans le groupe constitué par l'hydrolysat partiel d'amidon, l'amidon liquéfié, l'amidon gélatinisé et leurs mélanges.

8. Procédé selon l'une quelconque des revendications 3 à 7, dans lequel la concentration dudit saccharide α-glucosylique est 0,5 à 100 fois supérieure à celle de la naringine.

9. Procédé selon l'une quelconque des revendications 3 à 8, dans lequel ledit liquide est en suspension.

10. Procédé selon l'une quelconque des revendications 3 à 8, dans lequel ledit liquide est en solution.

11. Procédé selon la revendication 10, dans lequel on peut obtenir ledit liquide par dissolution de la naringine en chauffant.

12. Procédé selon la revendication 10, dans lequel on peut obtenir ledit liquide par dissolution de la naringine à un pH alcalin excédant 7,0.

13. Aliments et boissons, qui contiennent de la naringine α-glycosylique.

14. Aliments et boissons selon la revendication 13, où ladite naringine α-glycosylique est une naringine α-glucosylique.

15. Aliments et boissons selon la revendication 13, où ladite naringine α-glycosylique est préparée par le procédé de l'une quelconque des revendications 3 à 12.

16. Aliments et boissons selon l'une quelconque des revendications 13 à 15, qui contiennent en outre de la vitamine C.

17. Procédé de préparation d'un produit pharmaceutique pour des maladies susceptibles, lequel procédé comporte le mélange avec un porteur acceptable du point de vue pharmaceutique d'une naringine α-glycosylique comme ingrédient efficace.

18. Procédé selon la revendication 17, dans lequel ladite naringine α-glycosylique est une naringine α-glucosylique.

19. Procédé selon la revendication 17, dans lequel ladite naringine α-glycosylique est préparée par le procédé de l'une quelconque des revendications 3 à 12.

20. Procédé selon l'une quelconque des revendications 17 à 19, qui comporte en outre le mélange de vitamine C avec le support acceptable du point de vue pharmaceutique et/ou la naringine α-glycosylique.

21. Cosmétique qui contient comme ingrédient efficace une naringine α-glycosylique.

22. Cosmétique selon la revendication 21, dans lequel ladite naringine α-glycosylique est une naringine α-glucosylique.

23. Cosmétique selon la revendication 21, dans lequel ladite naringine α-glycosylique est préparée par le procédé de l'une quelconque des revendications 3 à 12.

24. Cosmétique selon l'une quelconque des revendications 21 à 23, qui contient en outre de la vitamine C.

25. Naringine α-glycosylique que l'on peut obtenir par le procédé de l'une quelconque des revendications 3 à 12.
